# EUROPÄISCHE PATENTANMELDUNG

(11) **EP 0 968 714 A1**
(43) Veröffentlichungstag der Anmeldung: **05.01.2000**
(21) Anmeldenummer: 98112241.9
(22) Anmeldetag: 02.07.1998
(51) Int. Cl.: A61K 31/40, A61K 9/00

(54) **Verfahren zur Herstellung schnellauflösender pharmazeutischer Zubereitungen von schwerlöslichen Wirkstoffen**

(71) Anmelder: Roche Diagnostics GmbH, 68298 Mannheim (DE)
(72) Erfinder: Gabel, Rolf-Dieter, Dr., 68723 Schwetzingen (DE); Preis, Walter, Dipl.-Che.Ing., 67433 Neustadt (DE); Wirl, Alexander, Dipl.-Che-Ing., 67259 Heuchelheim (DE)

(57) **Zusammenfassung**

Die Erfindung betrifft ein Verfahren zur Herstellung schnellauflösender pharmazeutischer Zubereitungen von schwerlöslichen Wirkstoffen, das dadurch gekennzeichnet ist, daß man aus dem Wirkstoff und einem oder mehreren wasserlöslichen Hilfsstoffen eine wäßrige Suspension herstellt und anschließend die erhaltene wäßrige Suspension unter Entfernung des Wassers nach an sich üblichen Verfahren zu festen pharmazeutischen Zubereitungen verarbeitet.

Daneben betrifft die Erfindung schnellauflösende pharmazeutische Zubereitungen von Wirkstoffen mit einer Auflösungsrate von mindestens 70 % nach 30 Minuten, die nach dem erfindungsgemäßen Verfahren hergestellt sind.

## Beschreibung

Die Erfindung betrifft ein Verfahren zur Herstellung schnellauflösender pharmazeutischer Zubereitungen von schwerlöslichen, zur Agglomeratbildung neigenden Wirkstoffen mit einer Auflösungsrate von mindestens 70% nach 30 Minuten und nach diesem Verfahren hergestellte pharmazeutische Zubereitungen.

Bei Arzneistoffen, die nicht im Magen-Darm-Bereich selbst ihre Wirkung entfalten sollen, sind die Freisetzung aus der Arzneiform im Gastrointestinaltrakt und die anschließende Resorption die notwendige Voraussetzung für einen therapeutischen Effekt. Problematisch sind dabei solche Wirkstoffe, die aufgrund ihrer schlechten Löslichkeit, beziehungsweise ihrer geringen Lösungsgeschwindigkeit, im wäßrigen Milieu oder aufgrund einer verhinderten Freisetzung aus dem System der übrigen Hilfsstoffe eine so geringe Konzentration im Magen-Darm-Trakt erreichen, daß die Auflösung des Wirkstoffs der geschwindigkeitsbestimniende Schritt bei der Resorption ist. Aufgrund der dadurch bewirkten geringen Resorptionsgeschwindigkeit erreichen solche Wirkstoffe dann keine ausreichende Bioverfügbarkeit. Als Problemarzneistoffe dieser Art werden normalerweise Wirkstoffe bezeichnet, die eine Löslichkeit von weniger als 5 g/l Wasser aufweisen oder deren Auflösungsrate aus einer festen Arzneiform nach 30 Minuten weniger als 50 % beträgt. Löslichkeit und Freisetzungsrate werden dabei nach genormten Methoden, beispielsweise gemäß der Paddle-Methode nach USP XXII bestimmt.

Da der Erhöhung der Löslichkeit durch die Natur der Wirkstoffe relativ enge Grenzen gesetzt sind (beispielhaft seien genannt Salzbildung, Derivatisierung mit löslichkeitsverbessernden Gruppen, die die Wirkung nicht beeinflussen oder die im Blut wieder abgespalten werden, Herstellung löslicher Solvate oder anderer Komplexe oder die Uberführung in energiereiche und damit besser lösliche Kristallformen), wurde in der Vergangenheit das Hauptaugenmerk auf die Erhöhung der Lösungsgeschwindigkeit gerichtet. Da nach den bekannten Fick'schen Gesetzen die Auflösungsgeschwindigkeit proportional der Oberfläche des Wirkstoffs, dem Konzentrationsgefälle des Wirkstoffs zwischen der Oberfläche der Teilchen und der Lösung sowie der Dicke des an den Teilchen haftenden Diffusionsfilms ist, bieten sich bei durch Wirkstoff- und Lösungsmedium gegebenen Diffusionskoeffizienten drei Ansatzpunkte für eine Erhöhung der Lösungsgeschwindigkeit.

Die Dicke der Diffüsionsschicht ist praktisch abhängig von der Bewegung der Wirkstoffteilchen im Magen-Darm-Trakt und damit relativ wenig zu beeinflussen. Der Erhöhung der Konzentrationsgradienten sind relativ enge Grenzen gesetzt, da man lediglich durch Zusatz von Sprengmitteln und Tensiden für eine möglichst rasche Verteilung der Wirkstoffteilchen über den zur Verfügung stehenden Gastro-Intestinalraum sorgen kann. Aus diesem Grund wird eine möglichst große Wirkstoffoberfläche erzeugt. So werden Wirkstoffe durch feines Vermahlen oder rasches Fällen in einen mikrokristallinen, beziehungsweise amorphen Zustand überführt bzw. durch Auflösen in der Schmelze oder Lösung eines leicht löslichen Hilfstoffes und anschließendes Erstarren beziehungsweise Verdampfen des Lösungsmittels eine molekular disperse, amorphe oder mikrokristalline Verteilung des Wirkstoffs in dem Hilfsstoff erreicht.

Es hat sich jedoch herausgestellt, daß die durch Vermahlung oder Fällen erhaltenen mikrokristallinen beziehungsweise amorphen Wirkstoffteilchen aufgrund ihrer sehr hohen Oberflächenenergie beim Verarbeiten, insbesondere unter Druck oder Zusatz von Lösungsmitteln, sowie bei längerer Lagerung zur Rekristallisation neigen, wodurch die Oberfläche und damit die Lösungsgeschwindigkeit in unkontrollierbarer Weise abnimmt. Darüber hinaus hat sich gezeigt, daß feine Teilchen dazu neigen, sich zu relativ festen Agglomeraten zusammenzulagern, die sich auch beim Einbringen in ein Lösungsmittel nur schwierig auseinanderbrechen lassen und sich deshalb wie ein entsprechend größeres Teilchen geringerer spezifischer Oberfläche verhalten. Deshalb werden solche Wirkstoffe zusammen mit den löslichen Hilfstoffen im Überfluß vermahlen, um so eine physikalische Trennung der Wirkstoffpartikel durch Trägerstoffpartikel zu erreichen. Auch durch einen erheblichen Überschuß der Hilfsstoffe läßt sich eine Rekristallisation beziehungsweise Agglomeration der Wirkstoffieilchen mit diesen Maßnahmen jedoch nicht vollständig verhindern, so daß die Auflösungsgeschwindigkeit solcher Präparate nicht optimal, vor allem aber nicht zeitunabhängig ist.

Die zweite Möglichkeit, fein verteilte Wirkstoffe zu erhalten, besteht darin, den Wirkstoff in einer Matrix eines hydrophilen, leicht löslichen Hilfsstoffes zu verteilen. Besonders bewährt haben sich in diesem Zusammenhang wasserlösliche Polymere, wie Polyvinylpyrrolidon, Polyethylenglycol und andere. Je nach den Eigenschaften des Wirkstoffs kann dies dadurch erfolgen, daß der Wirkstoff in einer Schmelze des Hilfsstoffs gelöst wird und diese entweder durch Sprüherstarren oder durch Vermahlen der erstarrten Schmelze zerteilt wird, worauf die erhaltenen Teilchen gegebenenfalls nach Vermischung mit weiteren Hilfstoffen in Granulate oder Tabletten verarbeitet werden. Soweit der Wirkstoff nicht ausreichend löslich ist beziehungsweise durch die Schmelztemperatur der Hilfsstoffe geschädigt wird, können beide Komponenten auch in einem geeigneten Lösungsmittel gelöst werden, aus dem sie bei Entfernung des Lösungsmittels als mehr oder weniger homogene Mischung zurückgewonnen werden. Nachteilig bei diesem Verfahren ist insbesondere, daß durch die Schwerlöslichkeit des Wirkstoffs in Wasser praktisch nur organische Lösungsmittel in Frage kommen, mit deren Verarbeitung die bekannten Probleme der Arbeitsplatzsicherung und Umweltbelastung verbunden sind.

Außerdem können aufgrund der Löslichkeitsverhältnisse nicht alle Wirkstoffe auf diese Weise verarbeitet werden, und die erhaltenen amorphen, beziehungsweise molekular dispersen Verteilungen des Wirkstoffes in der Hilfsstoffmatrix neigen zur Rekristallisation und damit zu einer Veränderung der Lösungsgeschwindigkeit der Wirkstoffe.

Der Erfindung lag die Aufgabe zugrunde, ein effizientes und umweltfreundliches Verfahren zur Herstellung schnellauflösender pharmazeutischer Zubereitungen von schwerlöslichen Wirkstoffen zu entwickeln, die normalerweise eine Auflösungsrate von weniger als 50 % nach 30 Minuten aufweisen und zur Agglomeratbildung beziehungsweise Rekristallisation neigen. Außerdem war es Aufgabe der Erfindung, schnell auflösende pharmazeutische Zubereitungen schwerlöslicher Wirkstoffe, wie z. B. Carvedilol, bereitzustellen.

Die erfindungsgemäße Aufgabe wird überraschend einfach gelöst durch Herstellen einer wäßrigen Suspension aus schwerlöslichem Wirkstoff und einem oder mehreren wasserlöslichen Hilfsstoffen und anschließender Verarbeitung dieser wäßrigen Suspension zu festen Formulierungen unter Entfernung des Wassers.

Gemäß der Erfindung werden schwerlösliche Wirkstoffe, wie z. B. Carvedilol, mit einer wäßrigen Lösung eines oder mehrerer geeigneter Hilfsstoffe vermischt und anschließend das Wasser abgetrieben. Dabei erwies es sich als besonders vorteilhaft, den Wirkstoff mit einem Teilchendurchmesser von weniger als 500 µm, vorzugsweise mit einem Teilchendurchmesser von weniger als 250 µm, besonders bevorzugt mit einem Teilchendurchmesser von weniger als 100 µm, einzusetzen. Der Wirkstoff wird dazu nach an sich bekannten Methoden mechanisch zerkleinert.

Hilfsstoffe sind im Sinne der Erfindung alle leicht wasserlöslichen, pharmazeutisch unbedenklichen und mit dem Wirkstoff nicht negativ reagierenden Stoffe. So werden alle an sich üblichen Binde-, Füll-, Spreng- und grenzflächenaktive Mittel (Netzmittel, Tenside) eingesetzt. Dabei kommen bevorzugt Mono- und Disaccharide, wie z.B. Saccharose, Glucose und Lactose; Oligo- und Polysaccharide, wie z. B. Stärke; Zuckeralkohole, wie z. B. Mannit und Sorbitol; gut wasserlösliche Cellulosederivate, wie z. B. Methylhydroxypropylcellulose; Polyvinylpyrrolidone und Polyethylenglycole zur Anwendung. Darüber hinaus sind alle weiteren bekannten pharmazeutischen Hilfsstoffe einsetzbar.

Leicht wasserlösliche Hilfsstoffe sind bevorzugt, da, je nach Löslichkeit des Hilfsstoffes, entsprechende Wassermengen wieder entfernt werden müssen. Zur Vermeidung eines großen Aufwandes bei der Entfernung des Wassers wird deshalb die Hilfsstoffmenge so gering wie möglich gehalten.

So liegt das Wirkstoff-HilfsstoflVerhältnis der Trockensubstanz in der Suspension im Bereich 1:0,01 bis 1:500, vorzugsweise im Bereich 1 : 0,1 bis 1:50, besonders bevorzugt bei 1 : 0,1 bis 1:10, in Abhängigkeit von der Formulierungsart und -größe und der Menge notwendiger und Trägerstoffe.

Gegebenenfalls werden der wäßrigen Suspension des schwerlöslichen Wirkstoffes grenzflächenaktive Mittel zugegeben, wobei das Verhältnis Wirkstoff zu grenzflächenaktivem Mittel im Bereich bis zu 1:1, vorzugsweise bis zu 1:0,3, in einer besonders bevorzugten Ausführungsform bis zu 1:0,05 liegen kann.

Als grenzflächenaktive Mittel werden sowohl ionische als auch nichtionische Tenside eingesetzt, wie z. B. Benzalkoniumchlorid, Polyoxyethylen-Polyoxypropylen-Copolymere (z. B. Pluronic F 68), Alkylsulfate, vorzugsweise Natrium-Dodecylsulfat und Stearate, wie Polyethylenglycol-400-stearat (Myrj).

Gemaß einer erfindungsgemäßen Ausführungsform wird ein grenzflächenaktives Mittel in Wasser gelöst und der schwerlösliche Wirkstoff wird zusammen mit einem oder mehreren Hilfsstoffen dieser Lösung zugemischt.

Darüber hinaus kann der wäßrigen Suspension aus Wirkstoff und Hilfsstoffen zusätzlich ein wasserunlöslicher Träger zugemischt werden, oder die wäßrige Suspension wird auf einen solchen wasserunlöslichen Träger aufgebracht. Dabei kann der Anteil des wasserlöslichen Trägers im Verhältnis zum Wirkstoff bis zu 50:1 betragen. In einer bevorzugten Variante wird der schwerlösliche Wirkstoff zusammen mit dem wasserunlöslichen Träger und gegebenenfalls zusammen mit weiteren wasserlöslichen Hilfsstoffen in eine wäßrige Hilfsstofflösung eingerührt.

Als wasserunlösliche Träger kommen vorzugsweise hochdisperses Siliciumdioxid oder Aluminiumoxid zur Anwendung. Vorzugsweise liegt der Anteil von hochdispersem Siliciumdioxid oder Aluminiumoxid bei bis zu 20 %, bezogen auf den festen Wirkstoff.

Die sich an die Zubereitung der Suspension anschließende Überführung dieser in feste pharmazeutische Zubereitungen erfolgt nach an sich üblichen Verfahren. So ist eine bevorzugte Variante die Sprühtrocknung, wodurch je nach Trocknergröße und Zerstäubungsart Pulver oder Granulate erhalten werden. Diese Pulver oder Granulate (Pulver gegebenenfalls nach vorheriger Granulierung) werden zu festen Arzneiformen wie, z. B. Tabletten, Dragees, Kapseln, Pellets oder Globuli weiterverarbeitet. Gegebenenfalls werden dabei weitere übliche Hilfsstoffe, wie z. B. Füllstoffe wie hydrophile Kohlenhydrate, wie z. B. Zucker, vorzugsweise Glucose, Lactose und Saccharose, z.B. Zuckeralkohole, wie Mannit und Sorbitol; z. B. Stärke und Stärkederivate; Bindemittel, wie z. B. Gelatine, mikrokristalline Cellulose, Polyvinylpyrrolidon-Derivate und L-HPC; Sprengmittel, wie z. B. Carboxymethylcellulose, Starch 1500 und Natrium-Carboxymethylstärke, ionische und nichtionische Tenside, Gleitmittel, wie z.B. Talkum oder Polyethylenglykole; Schmier- und Formtrennmittel, wie z. B. Magnesium- oder Calciumstearat, Stearinsäure, 1-Hexadecanol; Fließregulierstoffe, wie z.B. hochdisperses Siliciumdioxid, Talkum zugemischt.

In einer anderen Variante wird die wäßrige Suspension direkt zur Feuchtgranulierung, z. B. in der Wirbeischicht oder im Schnellmischer, gegebenenfalls mit den genannten üblichen Hilfsstoffen eingesetzt, und das entstandene Granulat wird in an sich bekannter Weise getrocknet und weiterverarbeitet. Durch das Verdunsten des Wassers überziehen sich zunächst die Wirkstoffpartikel mit einer Schicht aus den in der Suspension gelösten Hilfsstoffen. Zusätzlich werden diese überzogenen Partikel mit den in der Vorlage befindlichen Hilfsstoffen zu größeren Einheiten verbunden. Wenn ein im Verhältnis zur Hilfsstoffvorlage hohes Suspensionsvolumen vorliegt, führt man die Feuchtgranulierung vorteilhafterweise in mehreren Schritten durch, das heißt, es werden während der Granulierung Zwischentrocknungsschritte eingeschoben.

In einer weiteren Variante der Erfindung wird die wirkstoffhaltige Suspension auf Pellets oder Globuli aufgetragen oder zur Herstellung von Pellets verwendet.

Eine weitere Verfahrensvariante zur Herstellung fester pharmazeutischer Zubereitungen schwerlöslicher Wirkstoffe wird über Sprüherstarrung des Wirkstoffs aus einer Schmelze des oder der wasserlöslichen Hilfsstoffe realisiert, wobei diese Suspension als Variante auch einen hochdispersen Träger, wie z. B. Siliciumdioxid, enthalten kann.

Es hat sich gezeigt, daß der Wirkstoff in der erfindungsgemäß hergestellten und eingesetzten wäßrigen Suspension in der stabilen Ausgangskristallform vorliegt, die sich auch während der Verarbeitung nicht ändert, so daß Änderungen in der Kristallmodifikation bei den nach dem erfindungsgemäßen Verfahren hergestellten festen pharmazeutischen Zubereitungen der schwerlöslichen Wirkstoffe weitgehend auszuschließen sind. Das heißt, es finden keine signifikanten Umwandlungsprozesse bzw. unkontrollierte Rekristallisationen in andere Kristallmodifikationen während der Lagerung der Darreichungsformen statt. Die in der Suspension gelösten Hilfsstoffe fallen nach dem Trocknen in einem teil- oder vollamorphen Substanzgemisch an. Diese Struktur des Substanzgemisches bleibt auch bei Lagerung über Jahre weitgehend erhalten, was z. B. durch Röntgenbeugungsuntersuchungen bestätigt wird.

Das erfindungsgemäße Verfahren hat den Vorteil, daß weder die Verwendung organischer Lösungsmittel noch hohe Temperaturen notwendig sind.

Die erfindungsgemäß hergestellten festen pharmazeutischen Zubereitungen weisen eine überraschend hohe Auflösungsrate von mindestens 70 %, vorzugsweise mindestens 80 % nach 30 Minuten auf Insbesondere können mit dem erfindungsgemäßen Verfahren feste pharmazeutische Zubereitungen von Carvedilol und 4-[2-Hydroxy-3-[4-(phenoxymethyl)piperidino]-propoxy-indol, Acetate mit dieser Auflösungsrate hergestellt werden.

Im Vergleich dazu liegen die Auflösungsraten der Reinsubstanzen oder Pulver dieser Wirkstoffe mit hydrophilen Hilfsstoffen zum Teil weit unter 50 % nach 30 Minuten. Durch die hohe Neigung dieser Wirkstoffe, Agglomerate zu bilden, führt die Vergrößerung der Oberfläche durch Mahlung auch bei Zugabe hydrophiler Hilfsstoffe nicht zu einer signifikanten Verbesserung der Auflösungsgeschwindigkeit. Demzufolge weisen die mit den üblichen Methoden und mit üblichen pharmazeutischen Hilfsstoffen hergestellten Granulate, Tabletten, Kapseln ebenfalls unbefriedigende Wirkstoffauflösungsraten auf Auch bei Bereitstellung von Tabletten mit mikronisiertem Wirkstoffliegt die Auflösungsrate nach 30 Minuten unter 50 % (vgl. Beispiele 1 und 2).

Die erfindungsgemäß hergestellten Zubereitungen können auch als Basis für Zubereitungen mit modifizierter Freisetzung eingesetzt werden. Während z. B. bei herkömmlichen Retardformen mit schwerlöslichen Wirkstoffen die Wirkstoff-Freisetzung nicht nur von den retardierenden Hilfsstoffen, sondern mehr oder weniger auch vom Auflösungsverhalten der schwerlöslichen Wirkstoffe bestimmt wird, kann bei Verwendung der erfindungsgemäßen Zubereitungen eine Freisetzungssteuerung in alleiniger Abhängigkeit von den retardierenden Hilfsstoffen erreicht werden.

Anschließend soll die Erfindung an Beispielen näher erläutert werden.

### Beispiel 1: (Vergleichsbeispiel)

In-vitro-Auflösungsraten der Wirkstoffe 4-[2-Hydroxy-3-[4-(phenoxymethyl)piperidino]-propoxy-indol (Acetat-Form) (A) und Carvedilol (B) und beziehungsweise der Mahlungen mit hydrophilen Hilfsstoffen - in Pulverform.

| Rezeptur | mg | Verfahren | In-vitro-Auflösungsrate nach Min. in % | | | |
|---|---|---|---|---|---|---|
| | | | 10 | 20 | 30 | 60 |
| Wirkstoff A | | Reinsubstanz | 31 | 43 | 50 | 58 |
| Wirkstoff A | 80 | zusammen | 40 | 46 | 53 | 62 |
| Lactose D 80 | 60 | mikronisiert | | | | |
| Wirkstoff B | | Reinsubstanz | 36 | 46 | 49 | 56 |
| Wirkstoff B | 30 | zusammen | 20 | 26 | 27 | 29 |
| Saccharose | 30 | mikronisiert | | | | |
| Wirkstoff B | 30 | zusammen | 24 | 26 | 27 | 29 |
| Lactose D 80 | 30 | mikronisiert | | | | |

### Beispiel 2: (Vergleichsbeispiel)

### Auflösungsrate von Tabletten mit mikronisiertem Wirkstoff

Carvedilol/Lactose-Strahlmahlung wird mit weiteren hydrophilen Hilfsstoffen und Sprengmitteln wie Lactose, Poly(1-vinyl-2-pyrrolidon), quervernetzt und Poly-(1-vinyl-2-pyrrolidon) gemischt, mit einer Polyethylenstearat-Lösung (Myrj 52) granuliert, getrocknet und gesiebt. Das Granulat wird mit üblichen pharmazeutischen Hilfsstoffen, wie Poly(1-vinyl-2-pyrrolidon), quervernetzt, hochdispersem Siliciumdioxid und Magnesiumstearat gemischt und zu Tabletten verpreßt.

In-vitro-Auflösungsrate von Carvedilol nach Min. in %

| | | | | |
|---|---|---|---|---|
| 10 | 20 | 30 | 60 | min |
| 22 | 36 | 42 | 59 | % |

Die In-vitro-Auflösungsraten in diesem und den folgenden Beispielen wurden nach USP XXII, Paddle-Methode in wäßrigem Puffer pH 4,5 bestimmt

### Beispiel 3:

### Carvedilol-Suspension zur Sprübtrocknung

75 mg Myrj 52 werden in 700 g Wasser gereinigt, gelöst und anschließend 300 g Carvedilol, 300 g Saccharose und Hydroxypropylmethylcellulose mit einem schnellaufenden Rührer in die Lösung eingearbeitet. Die wäßrige Suspension wird sprühgetrocknet.

### In-vitro-Auflösungsrate

| | | | | |
|---|---|---|---|---|
| 10 | 20 | 30 | 60 | min |
| 73 | 81 | 83 | 86 | % |

### Beispiel 4:

### Carvedilol-Tabletten

69 g des nach Beispiel 3 sprühgetrockneten Produktes werden mit hydrophilen Hilfsstoffen (z. B. Lactose, Saccharose, Mannit etc.), Sprengmittel (z. B. Natriumcarboxymethylstärke, Poly(1-vinyl-2-pyrrolidon), quervernetzt, Maisstärke), hochdispersem Träger (Siliciumdioxid, hochdispers, Aluminiumoxid etc.) und Bindemittel Poly(1-vinyl-2-pyrrolidon) gemischt und mit Wasser granuliert. Das feuchte Granulat wird getrocknet, gesiebt und anschließend mit einem Formentrennmittel (gegebenenfalls Zusatz eines Fließmittels und/oder Sprengmittels) zu Tabletten mit einem Wirkstoffgehalt von 30 mg und einem Endgewicht von 180 mg verpreßt.

### In-vitro-Auflösungsrate aus den Tabletten

| | | | | |
|---|---|---|---|---|
| 10 | 20 | 30 | 60 | min |
| 81 | 88 | 96 | 98 | % |

### Beispiel 5:

### Carvedilol-Kapseln

Das nach Beispiel 3 sprühgetrocknete Produkt wird mit hydrophilen Hilfsstoffen, gegebenenfalls Fließmitteln, Sprengmitteln und Formentrennmitteln gemischt und aufherkömmlichen Kapselfüllmaschinen in Steckkapseln abgefüllt.

### In-vitro-Auflösungsrate aus der Kapselfüllmasse:

| | | |
|---|---|---|
| 10 | 20 | min |
| 95 | 100 | % |

### Beispiel 6

### Carvedilol-Granuliersuspension

75 g Myrj 52 werden in 700 g Wasser gereinigt, gelöst und anschließend 300 g Carvedilol und 300 g Saccharose mit einem schnellaufenden Rührer in die Lösung eingearbeitet.

### Beispiel 7

### Carvedilol-Tabletten

Die wäßrige Granuliersuspension nach Beispiel 6 wird auf eine Mischung von hydrophilen Hilfsstoffen, Sprengmittel, hochdispersem Träger und Bindemittel aufgezogen, getrocknet und gesiebt.

Unter Zusatz eines Formentrennmittels, gegebenenfalls auch eines Fließmittels und Sprengmittels, werden Tabletten mit einem Endgewicht von 180 mg mit einem Gehalt von 30 mg Carvedilol hergestellt.

### In-vitro-Auflösungsrate

| | | | | |
|---|---|---|---|---|
| 10 | 20 | 30 | 60 | min |
| 78 | 90 | 93 | 97 | % |

Die erfindungsgemäße Wirkstoffsuspension beziehungsweise die daraus hergestellten Sprühprodukte oder Granulate können ein Tensid (z. B. Polyoxyethylenstearat) in Form von Myrj 52 oder Myrj 53 enthalten. In der Suspension kann das Verhältnis von Wirkstoff zu Tensid im Bereich bis zu 1:1, vorzugsweise bis zu 1:0,3 liegen.

Gegebenenfalls wird der Hilfsstoff Hydroxypropyl-methylcellulose (Pharmacaot 603) in der Sprühtrocknungssuspension zur Verbesserung der Sprüh- und Produkteigenschaften zugesetzt.

### Beispiel 8:

### 4-[2-Hydroxy-3-[4-(phenoxymethyl)piperidino]-propoxy-indol, Acetate Suspension zur Sprühtrocknung

Der Wirkstoff 4-[2-Hydroxy-3-[4-(phenoxymethyl)piperidino]-propoxy-indol, Acetate wird in eine wäßrige Poly(1-vinyl-2-pyrrolidon)-Lösung zusammen mit einem hochdispersen Träger (z. B. hochdispersem Siliciumdioxid) und einem Sprengmittel (z. B. Poly(1-vinyl-2-pyrrolidon), quervernetzt, Primojel) eingerührt und homogenisiert.

Die wäßrige Suspension wird sprühgetrocknet.

### In-vitro-Auflösungsrate

| | | | | |
|---|---|---|---|---|
| 10 | 20 | 30 | 60 | min |
| 93 | 97 | 99 | 100 | % |

### Beispiel 9:

### 4-[2-Hydroxy-3-[4-(phenoxymethyl)piperidino]-propoxy-indol, Acetate Granuliersuspension

Der Wirkstoff wird in eine wäßrige Poly(1-vinyl-2-pyrrolidon)-Lösung zusammen mit einem hochdispersen Träger (z. B. hochdispersem Siliciumdioxid) und einem Sprengmittel (z. B. Poly(1-vinyl-2-pyrrolidon), quervernetzt, Primojel) eingerührt und homogenisiert.

Die wäßrige Granuliersuspension wird auf eine Mischung von hydrophilen Hilfsstoffen, Sprengmittel und hochdispersem Träger aufgezogen, getrocknet und gesiebt.

Das Aufbringen der Granuliersuspension auf die Hilfsstoffmischung erfolgt in einem üblichen Kneter, Granulator oder durch Aufsprühen im Wirbelbett.

### In-vitro-Auflösungsrate von 4-[2-Hydroxy-3-[4-(phenoxymethyl)piperidino]-propoxy-indol, Acetate aus Granulat

| | | | | |
|---|---|---|---|---|
| 10 | 20 | 30 | 60 | min |
| 71 | 88 | 94 | 97 | % |

### Beispiel 10:

### 4-[2-Hydroxy-3-[4-(phenoxymethyl)piperidino]-propoxy-indol, Acetate Tabletten und Kapseln

Sowohl die sprühgetrocknete Suspension nach Beispiel 8 als auch das Granulat nach Beispiel 9, erhalten durch Aufziehen der wäßrigen Granuliersuspension auf spezielle Hilfsstoffe, können nach an sich bekannten Methoden zu Tabletten, Filmtabletten, Dragees, Pellets, Hartgelatinekapseln Weichgelatinekapseln verarbeitet werden.

### In-vitro-Auflösungsrate von 4-[2-Hydroxy-3-[4-(phenoxymethyl)piperidino]-propoxy-indol, Acetate aus Tabletten:

| | | | | |
|---|---|---|---|---|
| 10 | 20 | 30 | 60 | min |
| 84 | 92 | 94 | 96 | % |

## Patentansprüche

1. Verfahren zur Herstellung schnellauflösender pharmazeutischer Zubereitungen von schwerlöslichen Wirkstoffen mit einer Auflösungsrate von mindestens 70 % nach 30 Minuten, dadurch gekennzeichnet, daß man aus dem Wirkstoff und einem oder mehreren wasserlöslichen Hilfsstoffen eine wäßrige Suspension herstellt und anschließend die erhaltene wäßrige Suspension unter Entfernung des Wassers nach an sich üblichen Verfahren zu festen pharmazeutischen Zubereitungen verarbeitet.

2. Verfahren nach Anspruch 1, dadurch gekennzeichnet, daß man als Wirkstoff Carvedilol oder 4-[2-Hydroxy-3-[4-(phenoxymethyl)piperidino]-propoxy-indol einsetzt.

3. Verfahren nach einem der Ansprüche 1 oder 2, dadurch gekennzeichnet, daß der Wirkstoff einen Teilchendurchmesser von weniger als 500 µm, vorzugsweise von weniger als 250, insbesondere weniger als 100 µm aufweist.

4. Verfahren nach einem der Ansprüche 1 bis 3, dadurch gekennzeichnet, daß man als wasserlösliche Hilfsstoffe an sich übliche Binde-, Füll-, Spreng- und/oder oberflächenaktive Mittel einsetzt.

5. Verfahren nach einem der Ansprüche 1 bis 4, dadurch gekennzeichnet, daß das Verhältnis Wirkstoff zu Hilfsstoff der Trockensubstanz in der Suspension 1 : 0,01 bis 1 : 500, vorzugsweise 1 : 0,1 bis 1 : 50, beträgt.

6. Verfahren nach einem der Ansprüche 1 bis 5, dadurch gekennzeichnet, daß man der wäßrigen Suspension ein oberflächenaktives Mittel zugibt, wobei das Verhältnis von Wirkstoff zu oberflächenaktivem Mittel im Bereich bis zu 1:1, vorzugsweise bis zu 1:0,3 liegt.

7. Verfahren nach einem der Ansprüche 1 bis 6, dadurch gekennzeichnet, daß man das oberflächenaktive Mittel in Wasser löst und anschließend den Wirkstoff zusammen mit einem oder mehreren Hilfsstoffen zumischt.

8. Verfahren nach einem der Ansprüche 1 bis 7, dadurch gekennzeichnet, daß man der wäßrigen Suspension einen wasserunlöslichen Träger zumischt oder die Suspension aufden Träger aufbringt.

9. Verfahren nach einem der Ansprüche 1 bis 8, dadurch gekennzeichnet, daß man den Wirkstoff zusammen. mit dem wasserunlöslichen Träger in eine wäßrige Hilfsstoffiösung einrührt, gegebenenfalls zusammen mit weiteren wasserlöslichen Hilfsstoffen.

10. Verfahren nach einem der Ansprüche 8 oder 9, dadurch gekennzeichnet, daß der wasserunlösliche Träger hochdisperses Siliciumdioxid oder Aluminiumoxid ist.

11. Verfahren nach einem der Ansprüche 1 bis 10, dadurch gekennzeichnet, daß man aus der wäßrigen 20 Suspension feste pharmazeutische Zubereitungen durch Sprühtrocknung herstellt.

12. Verfahren nach einem der Ansprüche 1 bis 10, dadurch gekennzeichnet, daß man aus der wäßrigen Suspension feste pharmazeutische Zubereitungen durch Feuchtgranulation herstellt, vorzugsweise in der Wirbelschicht oder im Schnellmischer.

13. Verfahren nach einem der Ansprüche 1 bis 10, dadurch gekennzeichnet, daß man aus dem in schmelzbaren Hilfsstoffen suspendiertem Wirkstoff feste pharmazeutische Zubereitungen durch Sprüherstarrung herstellt.

14. Schnellauflösende pharmazeutische Zubereitung eines schwerlöslichen Wirkstoffes mit einer Auflösungsrate von mindestens 70 % nach 30 Minuten, hergestellt nach einem der Ansprüche 1 bis 13, wobei der Wirkstoff in einem teil- oder vollamorphen Substanzgemisch eingebettet oder einem teil- oder vollamorphen Substanzgemisch umhüllt ist.

15. Pharmazeutische Zubereitung nach Anspruch 14, enthaltend Carvedilol als Wirkstoff.
